# EUROPEAN PATENT APPLICATION

(11) **EP 4 434 547 A1**
(43) Date of publication of application: **25.09.2024**
(21) Application number: 24165353.4
(22) Date of filing: 21.03.2024
(51) Int. Cl.: A61K 47/62, A61K 47/69, A61P 35/00

(54) **PREPARATION METHOD AND APPLICATION OF A TARGETED PEPTIDE MODIFIED NANODRUG DELIVERY SYSTEM (PDC) WITH SPECIFIC BINDING TO THE NEUROTENSIN RECEPTOR (NTR)**

(30) Priority: 24.03.2023 CN 202310296406
(71) Applicant: Shanghai Yizhong Pharmaceutical Co., Ltd, Shanghai, Shanghai 201403 (CN)
(72) Inventor: ZHOU, Jinsong, Shanghai, 201403 (CN); ZHANG, Wenming, Shanghai, 201403 (CN); SUN, Jing, Shanghai, 201403 (CN)
(74) Representative: Lermer, Christoph

(57) **Abstract**

A targeted peptide modified nano drug delivery system (PDC) that specifically binds to neurotensin receptor (NTR). The system is an amphiphilic block copolymer micelle specifically bind to the neurotensin receptors (NTR) through targeted peptide modification and the micelle is self-assembled through film hydration method, wherein the targeted peptide specifically binding to the neurotensin receptor (NTR) is composed of 14 amino acid sequences, with one end of the peptide being cysteine, which can specifically bind to the micelle; and the peptide can block a signaling pathway of the combination of neurotensin (NT) and NTR, and inhibit the biological function produced by the combination of NT and NTR. The targeted peptide modified nano drug delivery system that specifically binds to the neurotensin receptor (NTR) can be used for the preparation and development of anti-tumor drugs, which is of great significance for the clinical treatment of tumors.

## Description

### TECHNICAL FIELD

The present invention belongs to the field of biopharmaceutical technology and specifically relates to a drug loaded nano delivery system (PDC) that specifically binds to the neurotensin receptor (NTR) through targeted peptide modification, as well as its preparation method and application.

### BACKGROUND

Pancreatic cancer is the most malignant tumor of the digestive tract, with a very high mortality rate. Its five-year survival rate is less than 10%. Chemotherapy is the most effective treatment for pancreatic cancer besides surgery. However, even first-line chemotherapy drugs are far less effective than clinical expectations. Up to now, the treatment of pancreatic cancer has been facing serious challenges, and there is an urgent need to develop more efficient and less toxic novel technologies and treatments for it.

The targeted peptide modified drug delivery system of the present invention uses a peptide with 14 amino acid sequences (14P), which are analogues of neurotensin (NT) and can bind to the neurotensin receptors (NTR). NTR is highly tumor selective, and does not exist in normal pancreatic cells. About 75% of invasive pancreatic cancer overexpress NTR. The combination of NT and NTR plays corresponding biological functions and can activate multiple pathways closely related to cancer cell proliferation, migration, and invasion.

14P peptide, as an analogue of NT, blocks the signaling pathway of NT and NTR combination by binding to NTR, and inhibits the biological function produced by the combination of NT and NTR. The peptide can be conjugated with drugs to obtain peptide drug conjugates, or combined with carrier excipients, to encapsulate the drugs into liposomes or polymer micelles. The drugs can be chemotherapeutic agents or toxins, etc. The 14P peptide specifically binds to tumor cells expressing the neurotensin receptor (NTR), accurately and efficiently transporting the loaded drugs, to be applied effectively to the treatment of diseases related to the expression of the neurotensin receptor (NTR), such as pancreatic cancer.

Peptides are characterized of small molecular weight, simple structure, easy synthesis, strong specificity, high activity, low toxicity, and low immunogenicity, and are very advantageous to disease treatment. With the development of nanotechnology, the combination of nano drug carriers and peptides can fully leverage their advantages. Nanodrug carriers are drug delivery systems with a size within the nanoscale range, which can improve the bioavailability of drugs. For example, they can control the release process of drugs in the body, promote the crossing of biological barriers, and have a long half-life and different biological distribution characteristics. In addition, based on the high permeability and long retention effect, nanocarriers preferentially aggregate in tumor tissue, thereby reducing their toxicity to normal cells and enabling better treatment of tumors. Common nanocarriers used for drug delivery include organic nanocarriers such as liposomes, dendritic macromolecular polymer nanoparticles, polymer micelles, and inorganic nanocarriers such as metal, nanoparticles, carbon nanotubes, superparamagnetic iron oxide nanoparticles, etc.

Targeted peptide modified nano drug delivery systems, as a type of nano drug delivery system, can improve the accuracy and drug loading capacity of targeting tumor cells, thereby improving the efficacy of tumor treatment. They are drug innovation in drug delivery systems and disease treatment. However, up to now, there is no targeted peptide modified nano drug delivery system for the treatment of pancreatic cancer.

### SUMMARY

The purpose of the present invention is to provide a targeted peptide modified nano drug delivery system (PDC) that specifically binds to neurotensin receptor (NTR), and its preparation method and application as well. The targeted peptide modified nano drug delivery system that specifically binds to the neurotensin receptor (NTR) can be used for the preparation and development of anti-tumor drugs, which is of great significance for the clinical treatment of tumors.

To achieve the above objectives, the first aspect of the present invention provides a targeted peptide modified nano drug delivery system, wherein the targeted peptide modified nano drug delivery system formulation is composed of polyethylene glycol polylactide copolymer, peptides specifically bound to the neurotensin receptor (NTR) and the tumor therapeutic toxic drugs. Among them, there are three types of polyethylene glycol polylactide copolymers, one of which is formed by the combination of polyethylene glycol as a hydrophilic block through polymerization reaction with polylactide as a hydrophobic block; another is formed by the combination of a polyethylene glycol carrying a maleimide terminal group as a hydrophilic block is formed by combining with a polylactide as a hydrophobic block through a polymerization reaction; another copolymer formed by the combination of polyethylene glycol as a hydrophilic block and polylactide as a hydrophobic block through polymerization reaction, and then connected to Fmoc lysine through reaction; and the peptide can specifically bind to the neurotensin receptor (NTR), targeting pancreatic tumor cells overexpressing NTR. Preferably, the targeted peptide modified nano drug delivery system is in solution form or freeze-dried powder form.

According to the targeted polypeptide modified nano drug delivery system in the first aspect of the invention, the tumor treatment drug for pancreatic cancer is selected from one or more of the following drugs: paclitaxel, fluorouracil and their derivatives, gemcitabine, irinotecan and other drugs.

Preferably, the toxic drugs for tumor treatment are paclitaxel and gemcitabine.

According to the targeted polypeptide modified nano drug delivery system in the first aspect of the invention, the nano particle is selected from one or two of the following: liposomes and polymer micelles.

According to the targeted polypeptide modified nano drug delivery system in the first aspect of the invention, the neurotensin receptor (NTR), consisting of 14 amino acid sequences, with one end of the peptide being cysteine, which can specifically bind to polymer micelles. The peptide can block the signaling pathway of NT and NTR binding and inhibit the biological function produced by NT and NTR binding.

Preferably, the targeted peptide modified nanocarrier delivery system is a nanocarrier system formed by self-assembly of polyethylene glycol polylactide copolymer or polyethylene glycol polylactide Fmoc lysine copolymer and maleimide polyethylene glycol polylactide copolymer with tumor therapeutic drugs through thin film hydration method, which reacts with the targeted peptide to form a targeted peptide modified nanocarrier delivery system.

According to the targeted polypeptide modified nano drug delivery system in the first aspect of the invention, molecular weights of the three polyethylene glycol polylactide copolymers range from 1000 to 20000, preferably from 1000 to 15000, and further preferably from 1000 to 12000;

The particle size of the polymer micelles of the targeted peptide modified nano drug delivery system formulation is 10-70nm, preferably 10-50nm, and more preferably 10-30nm.

The molar ratio of the peptide, copolymer, and tumor therapeutic drug is 1:3:1-4:12-8:7-13.

The second aspect of the present invention provides a preparation method for a targeted peptide modified nano drug delivery system as described in the first aspect. The method comprises the following steps:
(1) prepare polyethylene glycol polylactide copolymer, polyethylene glycol polylactide Fmoc lysine copolymer, and maleimide polyethylene glycol polylactide copolymer separately;
(2) the copolymer and tumor therapeutic toxic drugs were self-assembled into nano polymer micelles through film hydration method;
(3) the above nano polymer micelles react with peptides to obtain targeted peptide modified nano drug loaded polymer micelle formulations;
(4) preferably, the targeted peptide modified nano drug loaded polymer micelle solution obtained in step (3) is dialyzed and filtered.

A preparation method according to a second aspect of the present invention, wherein in the step (1):
the catalyst used for preparing the copolymer is stannous octanoate, with a catalyst ratio of 0.5 ‰ -10 ‰ of the feed amount; the solvents used include dichloromethane, ethanol, ether, tetrahydrofuran, triethylamine, and pivaloyl chloride;
the molar ratio of polyethylene glycol to lactide used for preparing the copolymer is 1:10-25, preferably 1:10-20, and further preferably 1:12-18;
the polymerization reaction temperature is 100-150 °C, preferably 110-140 °C, and further preferably 125-135 °C;
the polymerization reaction time is 5-40 hours, preferably 5-35 hours, and further preferably 5-30 hours;
the refining temperature of the copolymer is -5 to -25 °C, preferably -10 to -25 °C, and further preferably -15 to -22 °C;
the drying time of the copolymer is 12-72 hours, preferably 15-60 °C, and further preferably 15-50 °C;
the molecular weight of the copolymer is 1000-20000, preferably 1000-15000, and further preferably 1000-12000.

A preparation method according to a second aspect of the present invention, wherein in the step (2):
the solvent for drug dissolution is acetonitrile or ethanol, preferably ethanol;
the hydration solvent is one or more of the following: phosphate buffer, ultrapure water, physiological saline, preferably phosphate buffer and ultrapure water;
the concentration of tumor therapeutic drugs in the micelle solution is 1-20mg/mL, preferably 1-15mg/mL, and further preferably 2-10mg/mL;
the concentration of polyethylene glycol polylactide copolymer or polyethylene glycol polylactide Fmoc lysine copolymer is 10-60mg/mL, preferably 15-50mg/mL, and further preferably 20-40mg/mL;
the concentration of maleimide polyethylene glycol polylactide copolymer is 0.5-25mg/mL, preferably 0.5-20mg/mL, and further preferably 1-15mg/mL;
the temperature of the hydration water bath is 35-65 °C, preferably 38-58 °C, and further preferably 42-55 °C;
the hydration time is 8-50 minutes, preferably 10-45 minutes, and further preferably 10-40 minutes.

A preparation method according to a second aspect of the present invention, wherein in the step (3):
the concentration of 14P peptide is 0.2-1.5mg/mL, preferably 0.2-1.mg/mL, further preferably 0.3-0.7mg/mL;
the reaction water bath temperature is 15-45 °C, preferably 16-40 °C, and further preferably 18-35 °C;
the reaction time is 3-24 hours, preferably 5-15 hours, and further preferably 5-12 hours;
According to the preparation method of the second aspect of the present invention, wherein in the step (4), the molecular weight of the dialysis membrane is 2000-10000Da, preferably 2000-7000Da, and further preferably 2000-5000Da; The pore size of the filter membrane is 0.2-1.0 µ m. Preferably 0.2 to 0.5 µ m, further optimized as 0.2-0.45 µ m, and the most preferably 0.22 µ M.

The third aspect of the present invention provides a targeted peptide modified nano drug delivery system as described in the first aspect or a targeted peptide modified nano drug delivery system prepared according to the method described in the second aspect for the application of cancer drugs:
preferably, the cancer is pancreatic cancer;
more preferably, the pancreatic cancer represents a pancreatic cancer cell or pancreatic cancer tissue with NTR expression or overexpression.

According to the application of the third aspect of the invention, the type of pancreatic cancer cells is selected from one or more of the following:
CFPAC-1, JF-305, BxPC 3, PANC 1, Capan 1, MiaPaCa 2, SW1990, HPAFII, and preferably SW1990 or HPAFII.

According to a particularly preferred embodiment of the invention, a nano drug delivery system capable of modifying targeted polypeptides targeting NTR targets is provided. The said polypeptide brings tumor cytotoxic drugs to pancreatic cancer tissues by combining with NTR, and achieves the purpose of treating pancreatic cancer by influencing tumor cell growth, proliferation, differentiation, metastasis, etc.

The targeted peptide modified nano drug delivery system of the present invention can have, but is not limited to, the following beneficial effects:
(1) The targeted peptide modified nano drug delivery system improves the solubility and biological stability of tumor toxic drugs, improves the targeting and binding ability of targeting pancreatic tumor tissue, and thus improves the ability to inhibit the proliferation, growth and metastasis of pancreatic cancer cells.
(2) The targeted peptide modified nano drug delivery system was shown by transmission electron microscopy to have a spherical structure with uniform particle size. The prepared micelles had a particle size distribution of 10-35nm, with an average particle size of 15-25nm.
(3) The targeted peptide modified nano drug delivery system can inhibit the proliferation of pancreatic cancer cells. When the tumor toxic drug is 20 mg/kg, it can significantly inhibit the proliferation of pancreatic cancer cells (Figure animal efficacy test).

### BRIEF DESCRIPTION OF THE DRAWINGS

In order to provide a clearer explanation of the specific embodiments of the present invention or the technical solutions in the prior art, the accompanying drawings required in the description of the embodiments or prior art are briefly introduced. It is evident that the accompanying drawings in the following description are some embodiments of the present invention. For ordinary technical personnel in the art, other accompanying drawings can be obtained based on these drawings without creative labor.
Figure 1 shows the nuclear magnetic resonance spectrum of polyethylene glycol polylactide copolymer in Example 1.
Figure 2 shows the magnetic spectrum of the core of maleimide polyethylene glycol poly (lactide) copolymer in Example 1.
Figure 3 shows the nuclear magnetic resonance spectrum of polyethylene glycol polylactide Fmoc lysine copolymer in Example 1.
Figure 4 shows the electron microscope image of the nano paclitaxel polymer micelle modified with targeted peptides in Example 2.
Figure 5 shows the particle size profile of nano paclitaxel polymer micelles modified with targeted peptides in Example 3.
Figure 6 shows the high-performance liquid chromatography of the targeted peptide modified nano paclitaxel polymer micelle content detection in Example 4.
Figure 7 is the result diagram of MTT cell proliferation detection method provided by Embodiment 5 of the invention to detect the effect of targeted polypeptide modified nano drug loaded polymer micelles on pancreatic cancer cell proliferation.
Figure 8 is the result diagram of the effect of targeted peptide modified nano drug loaded polymer micelles on pancreatic cancer cell apoptosis detected by AnnexinV staining and flow cytometry provided by Example 5 of the invention.
Figure 9 is a columnar schematic diagram of the effect of targeted peptide modified nano drug loaded polymer micelles on pancreatic cancer cell apoptosis detected by AnnexinV staining and flow cytometry provided by Example 5 of the invention.
Figure 10 is the result diagram for detecting the inhibition of targeted polypeptide modified nano drug loaded polymer micelles on pancreatic cancer cell migration through cell scratch test provided by Example 6 of the invention.
Figure 11 is a columnar schematic diagram of the results of inhibiting the migration of pancreatic cancer cells by detecting the targeted polypeptide modified nano drug loaded polymer micelles through the cell scratch test provided by Example 6 of the invention.
Figure 12 is the result diagram of the inhibition of targeted peptide modified nano drug loaded polymer micelles on the invasion of pancreatic cancer cells through the transwell invasion test provided by Example 6 of the invention.
Figure 13 is a columnar schematic diagram of the invasion result of targeted polypeptide modified nano drug loaded polymer micelles on pancreatic cancer cells detected through the transwell invasion experiment provided by Example 6 of the invention.
Figure 14 is the result diagram of detecting the inhibition of targeted polypeptide modified nano drug loaded polymer micelles on the growth of pancreatic cancer cells through cloning formation experiment provided by Example 7 of the invention.
Figure 15 is a columnar schematic diagram of the results of detecting the inhibition of targeted polypeptide modified nano drug loaded polymer micelles on the growth of pancreatic cancer cells through cloning formation experiment provided by Example 7 of the invention.
Figure 16 shows the results of the in situ tumor volume curve provided in Example 8 of the present invention.

### DETAILD DESCRIPTION

The present invention will be further explained in conjunction with the accompanying drawings and specific embodiments, but it should not be understood as a limitation of the present invention.

Without departing from the spirit and essence of the present invention, any modifications or substitutions made to the methods, process steps, and conditions of the present invention belong to the scope of the present invention. The experimental methods without specifying specific conditions and the reagents without specifying formulas in the implementation examples are based on conventional conditions in this field.

### Example 1

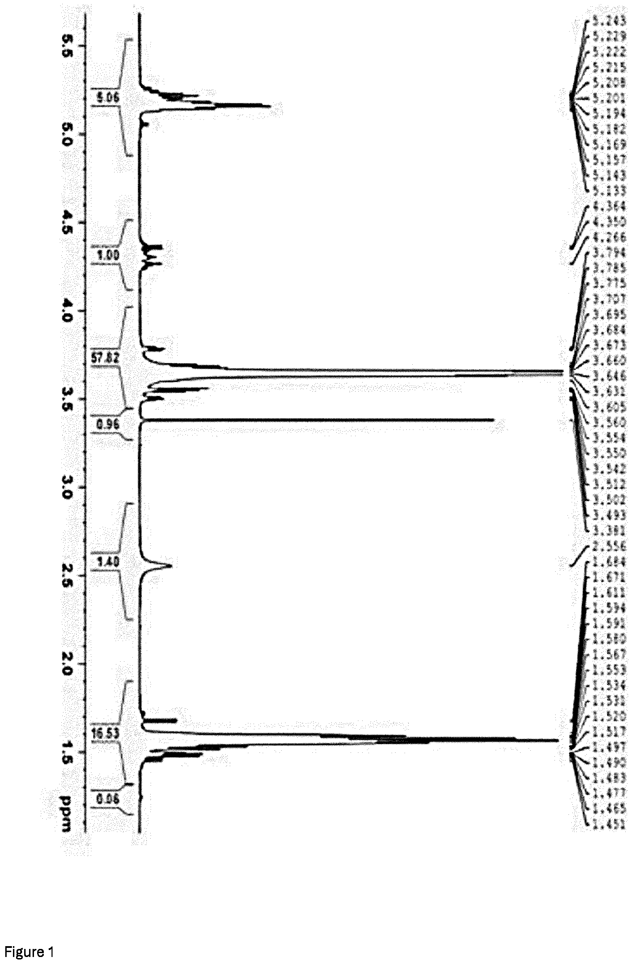

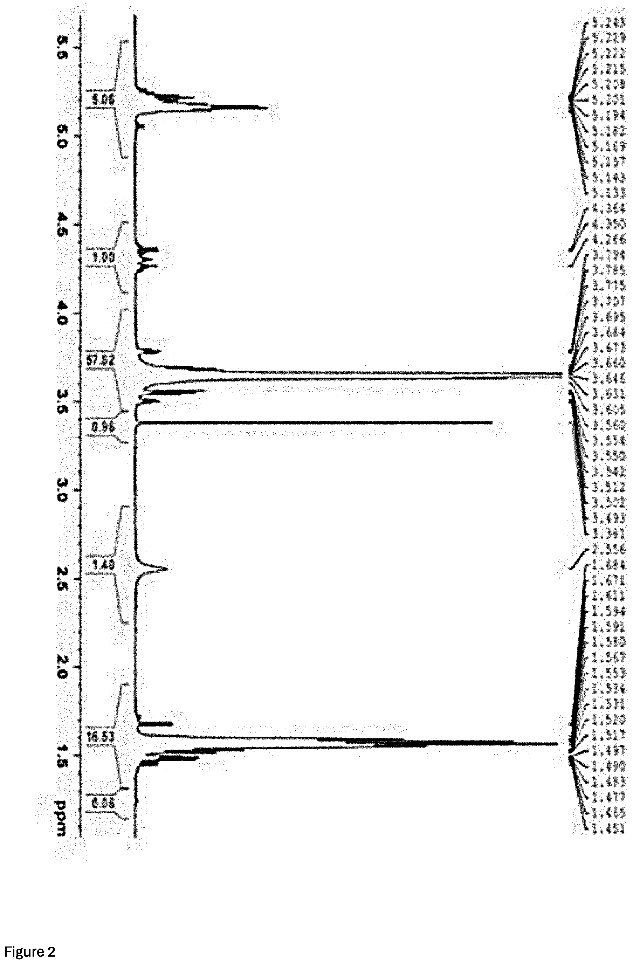

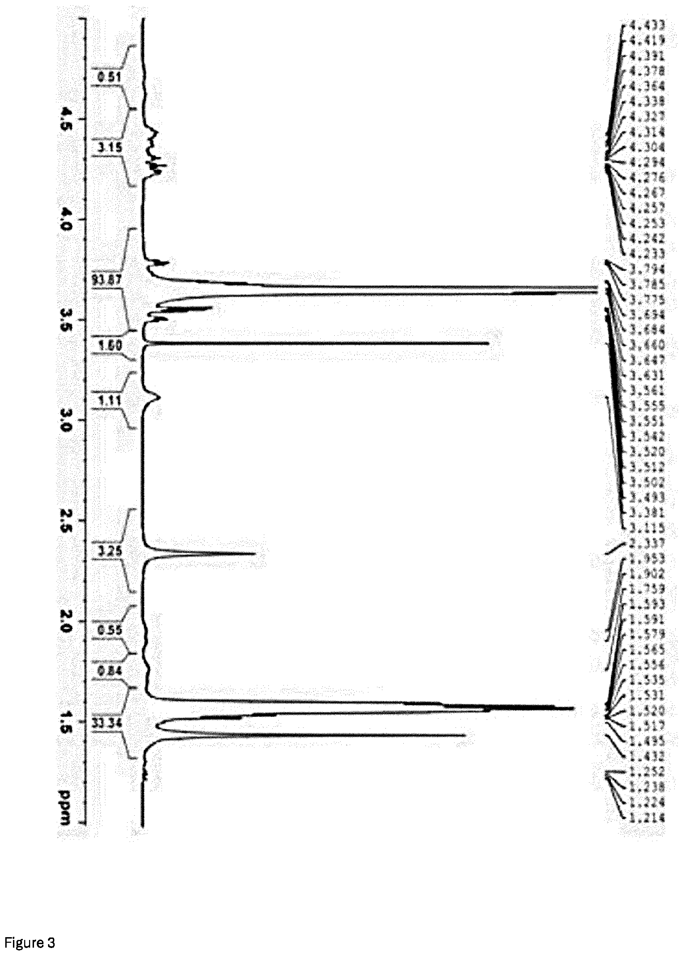

This embodiment is used to illustrate the preparation method of a targeted peptide modified nano drug delivery system of the present invention.
(1) Preparation method of polyethylene glycol polylactide copolymer or maleimide polyethylene glycol polylactide copolymer: a certain amount of polyethylene glycol or maleimide polyethylene glycol is polymerized with a certain amount of lactide under the action of catalyst stannous octanoate, and the obtained melt is refined 3-4 times in ice ether or ethanol, and dried to obtain. The copolymer nuclear magnetic resonance detection is shown in Figure 1 and Figure 2.
(2) Preparation method of polyethylene glycol polylactide Fmoc lysine copolymer: Fmoc lysine is dissolved in tetrahydrofuran, reacted with a certain amount of triethylamine and pivaloyl chloride for 2-4 hours, and the solution is dissolved in dichloromethane solution after rotary evaporation for standby; At the same time, a certain amount of polyethylene glycol polylactide is dissolved in a dichloromethane solution, followed by the addition of triethylamine and 4-pyrrolidine. Then, the aforementioned Fmoc lysine dichloromethane is dissolved and added, and the reaction is carried out at room temperature for 15-36 hours. The solution is then evaporated and refined with ice ethanol 3-4 times, and dried to obtain the product. The copolymer nuclear magnetic resonance detection is shown in Figure 3.
(3) Preparation method of tumor toxic drug nano micelles: Dissolve a certain proportion of tumor toxic drug paclitaxel, polyethylene glycol polylactide copolymer, polyethylene glycol polylactide Fmoc lysine copolymer, maleimide polyethylene glycol polylactide copolymer in ethanol or acetonitrile, completely dissolve, and then spin evaporate under vacuum conditions in a rotary evaporator to remove the solvent. Then add an appropriate amount of PBS buffer, Hydrate in a water bath at 42-55 °C for 10-40 minutes to obtain.
(4) Preparation of targeted peptide modified nano paclitaxel polymer micelles: Add a certain amount of 14P peptide powder to the micelle hydration solution in step 4 above, react under room temperature water bath conditions for 5-12 hours, dialyze with a dialysis bag for 10-20 hours, and add a certain amount of mannitol to completely dissolve.
(5) Apply 0.22 to the targeted peptide modified nano paclitaxel polymer micelle solution obtained from step (4) µ M filter membrane filtration, with 5ml of filtrate divided into 20ml penicillin bottles for freeze-drying.

### Example 2

Morphology analysis of targeted peptide modified nano paclitaxel polymer micelles. Transmission electron microscopy was used to characterize the morphology of targeted peptide modified nano paclitaxel polymer micelles.

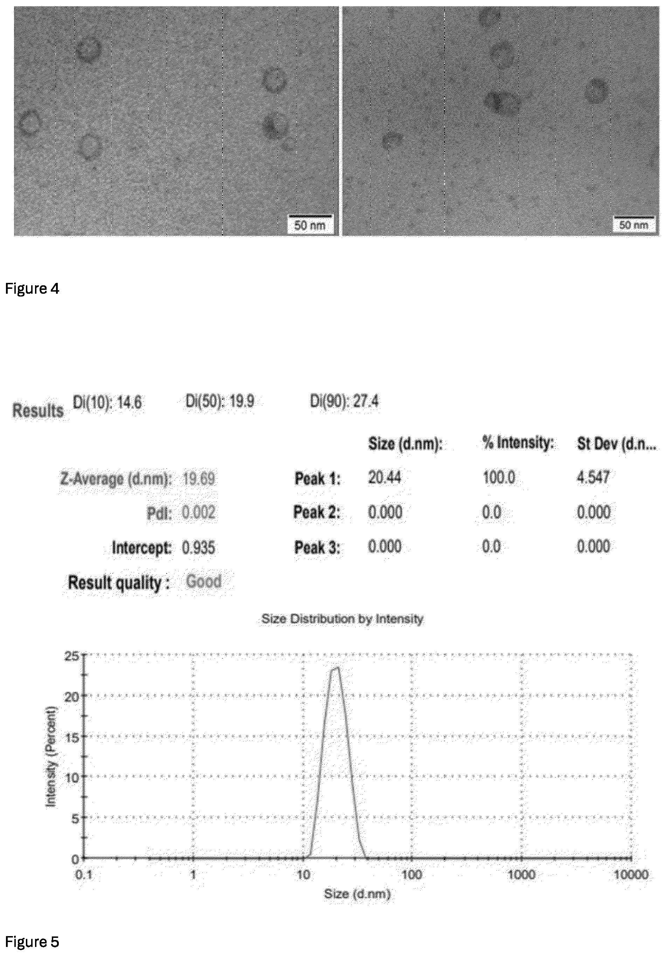

Release to 20 µ M. Drop 10 µ L liquid is placed on a copper mesh, left to stand overnight, and a 2wt% solution of uranyl acetate is added dropwise. After staining for 1 minute, the liquid is sucked up with filter paper and allowed to dry naturally. Then, it is observed and photographed under a transmission electron microscope. As shown in Figure 4, the size of drug loaded nano micelles is around 20nm.

### Example 3

Particle size analysis of targeted peptide modified nano paclitaxel polymer micelles. The particle size of targeted peptide modified nano paclitaxel polymer micelles was characterized using a particle size analyzer. Take one bottle of nano paclitaxel polymer micelles modified with targeted peptides, open the rubber stopper, and use a 10ml pipette to transfer 5ml of 0.9% sodium chloride injection along the bottle wall into a container for dissolution, as the test substance. Use a 1ml pipette to transfer 0.5ml of the test sample into an EP tube, add 2ml of 0.9% sodium chloride injection, shake it upside down 10 times, and place 1ml in a clean and scratch free particle size colorimetric cup. Cover the cup tightly as the test solution and place it in the sample tank for measurement. Take another bottle and operate the same method. Measure each bottle three times, repeat twice, and take the average value. As shown in Table 1, the average particle size of targeted peptide modified nano paclitaxel polymer micelles was measured to be 19-21nm, with a PDI<0. 1. The particle size detection graph is shown in Figure 5.

**Table 1**

| Samples | Z-Ave | Pdl | D(i)10 | D(i)50 | D(i)90 |
|---|---|---|---|---|---|
| | d.nm | | | | |
| Sample1-1 | 1957 | 0.011 | 145 | 19.9 | 27.4 |
| Sample1-2 | 19.58 | 0.003 | 14.3 | 19.9 | 276 |
| Sample1-3 | 1964 | 0.003 | 146 | 199 | 27.3 |
| Sample1-4 | 1986 | 0.006 | 14.7 | 20.1 | 27.7 |
| Sample1-5 | 19.66 | 0.005 | 14.5 | 199 | 27.5 |
| Sample1-6 | 19.69 | 0.002 | 14.6 | 19.9 | 27.4 |
| Sample2-1 | 20.25 | 0.020 | 15.0 | 20.6 | 28.2 |
| Sample2-2 | 202 | 0.013 | 15.0 | 20.5 | 28.0 |
| Sample2-3 | 20.06 | 0.024 | 15.0 | 20.4 | 27.9 |
| Sample2-4 | 20.41 | 0.006 | 15.2 | 20.7 | 28.2 |
| Sample2-5 | 20.18 | 0.011 | 15.0 | 20.5 | 28.0 |
| Sample2-6 | 2006 | 0.007 | 14.8 | 20.3 | 279 |

### Example 4

Analysis of the content of paclitaxel in nano paclitaxel polymer micelles modified with targeted peptides.

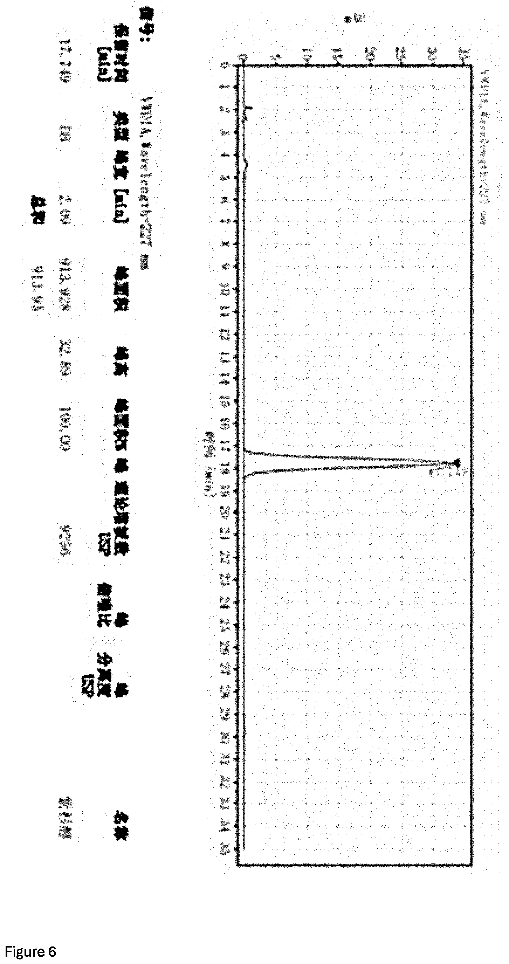

High performance liquid chromatography was used to characterize the content of paclitaxel in nano paclitaxel polymer micelles modified with targeted peptides. Accurately weigh approximately 0.1g of the freeze-dried sample into a 25mL volumetric flask, add an appropriate amount of 0.9% sodium chloride solution to dissolve, then dilute with acetonitrile to the mark and shake well. Centrifuge for 10 minutes (10000rpm) to take 1mL of the supernatant and place it in a 10m volumetric flask (prepare 2 parts). Dilute with acetonitrile to the mark, shake well, and filter to obtain the test solution. Take acetonitrile, paclitaxel system suitability solution, and paclitaxel reference solution (200.4982 µ G/mL), the test solution was tested sequentially and the graph data was recorded. It was found that the content of drug loaded nano micelles was 27.9mg/tube, which was 93% of the labeled amount. The HPLC spectrum for content detection is shown in Figure 6.

### Example 5

### Cell proliferation and apoptosis experiment

The cell proliferation experiment in this embodiment is as follows: take pancreatic cancer cells growing in logarithmic phase, inoculate them into 96 well plates, 5 × 103 pancreatic cancer cells. After 24 hours of cultivation in a 37 °C cell incubator, nano paclitaxel polymer micelles modified with different concentrations of targeted peptides were added. After further cultivation for 48 hours, add 20 µ Incubate the Aqueous One Solution of L in culture for about 1 hour, and then measure the absorbance value at 490nm using an enzyme-linked immunosorbent assay.

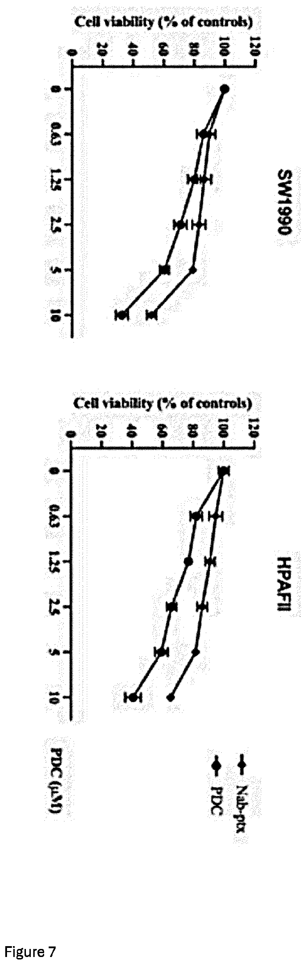

The results are shown in Figure 7. It can be seen from Figure 7 that after 48 hours of treatment of two different pancreatic cancer cells SW1990 and HPAFII with targeted polypeptide modified nano paclitaxel polymer micelles, the proliferation of the two pancreatic cancer cells was inhibited in a concentration gradient dependent manner, indicating that the targeted polypeptide modified nano paclitaxel polymer micelles can effectively inhibit the growth of cancer cells.

The cell apoptosis experiment operation in this embodiment is as follows:
(a) SW1990 cells were treated with nano paclitaxel polymer micelles or albumin bound paclitaxel (Nab PTX) modified with different concentrations of targeted peptides for 48 hours; After 48 hours, digest the cells. Centrifuge at 800rpm for 5 minutes and pour out the culture medium.
(b) Wash cells twice with PBS and use 0.1mL of 1 × Resuspension cells with binding buffer (BDBioscience) and incubate with PI and Annexin V in dark for 30 minutes.
(c) Add approximately 0.4mL of 1 to each tube × Binding buffer, filtered with a 400 mesh filter, and analyzed for cell apoptosis using flow cytometry.

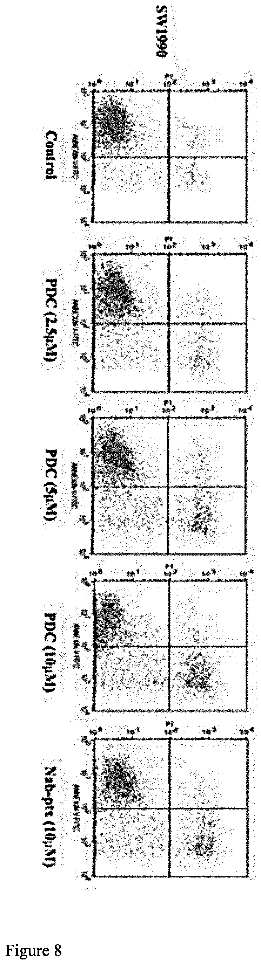

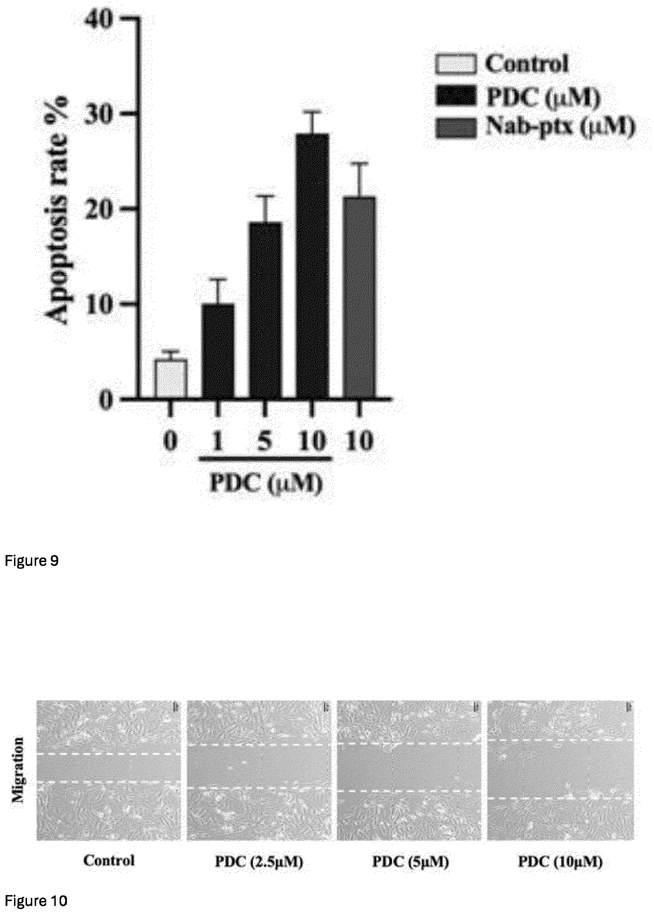

The results are shown in Figure 8 and Figure 9. It can be seen from Figure 8 and Figure 9 that the proliferation of pancreatic cancer cells sw1990 was inhibited in a concentration gradient dependent manner 48 hours after the targeted polypeptide modified nano paclitaxel polymer micelles treated pancreatic cancer cells sw1990, indicating that the targeted polypeptide modified nano paclitaxel polymer micelles can effectively inhibit the growth of cancer cells.

### Example 6

### Cell scratch detection cell migration experiment

The operation of the cell wound healing experiment in this embodiment is as follows: Take logarithmic tumor cells and inoculate them into a six well plate. When the cells grow to 95% density, use 100 µ L's gun head made a "ten" scratch on a six well plate filled with cells. Then wash twice with PBS and add 1.5mL of culture medium containing nano paclitaxel polymer micelles modified with different concentrations of targeted peptides. After the cells in the control group have fully migrated, terminate the experiment. Fix with 4% paraformaldehyde, take photos under an inverted microscope, and count the number of migrated cells using a mouse counter.

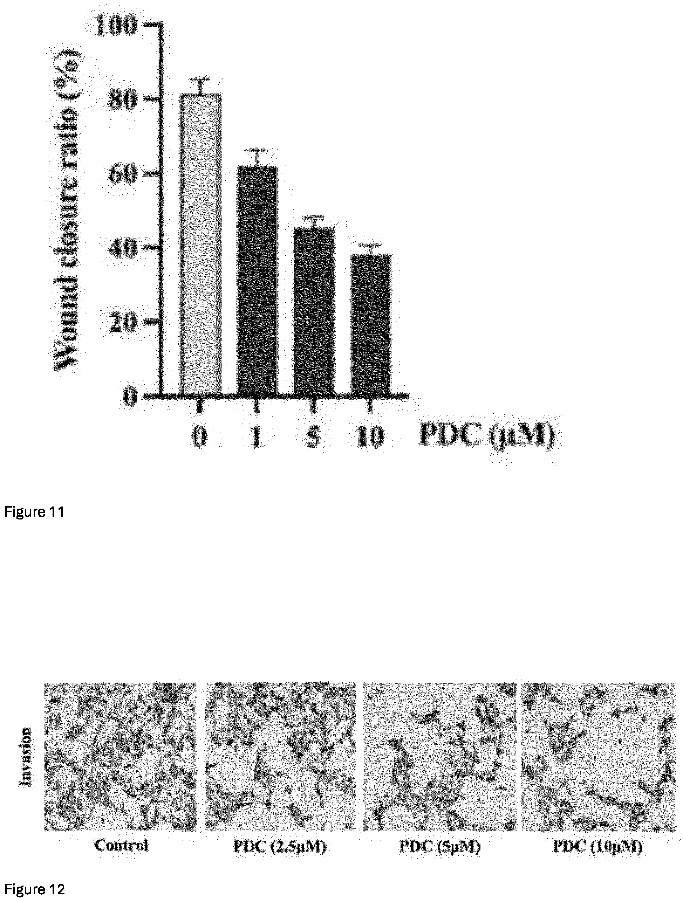

The results are shown in Figure 10 and Figure 11. It can be seen from the figure that the targeted peptide modified nano paclitaxel polymer micelles have an inhibitory effect on the migration of pancreatic cancer cells.

This embodiment utilizes a transwell cell with an aperture of 8 microns that can be placed in a 24 well plate, which is divided into an upper chamber and a lower chamber. The specific experimental method is as follows: Take logarithmic phase growing cells, digest them, count them, and prepare cell suspensions containing nano paclitaxel polymer micelles modified with different concentrations of targeted peptides. Take 100 cells each µ Add L to the upper chamber and 600 to the lower chamber µ L contains targeted peptide modified nano paclitaxel polymer micelles corresponding to the drug concentration in the upper chamber. Incubate for about 10 hours in a 37 °C cell incubator, remove the small chamber, wipe off non invading tumor cells from the upper chamber with a cotton swab, and fix at room temperature with paraformaldehyde for 15 minutes. Wash twice with PBS and stain with 0.1% crystal violet. Take photos and count under an inverted microscope.

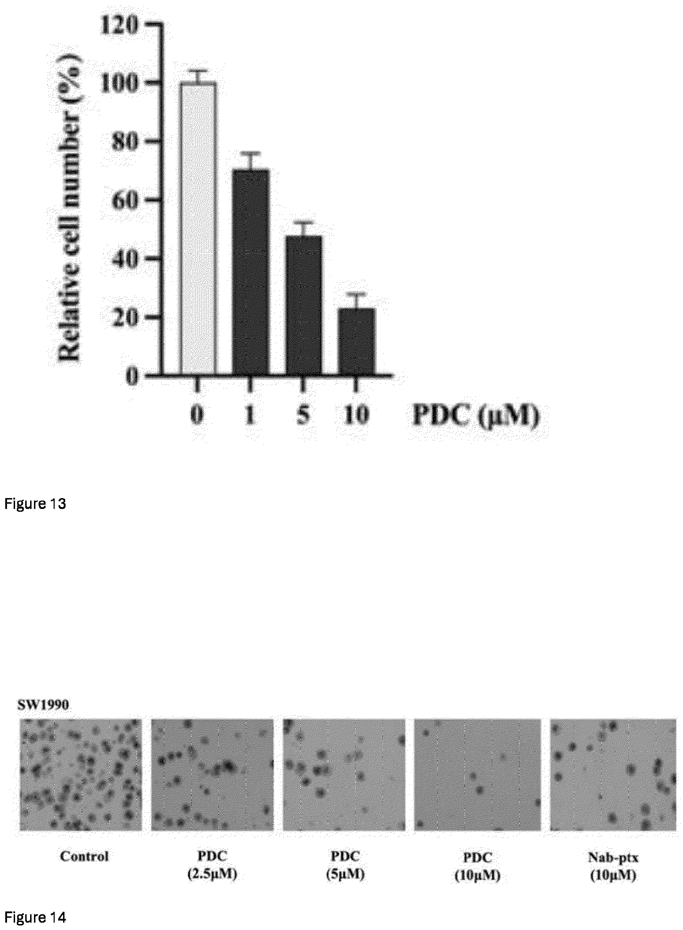

The results are shown in Figure 12 and Figure 13. It can be seen from the figure that the concentration gradient treatment of targeted peptide modified nano paclitaxel polymer micelles can significantly inhibit the ability of pancreatic cancer cells to invade their cells.

### Example 7

### Cell Clone Formation Experiment

The specific operation is as follows:
(a) Inoculate tumor cells (approximately 10³) into a 6-well plate.
(b) After 24 hours of cultivation, replace the fresh culture medium and add different concentrations of targeted peptide modified nano paclitaxel polymer micelles or albumin bound paclitaxel (Nab PTX) to the corresponding wells.
(c) After 8 days of cultivation, the clones were fixed with 4% PFA and then stained with 0.1% crystal violet.

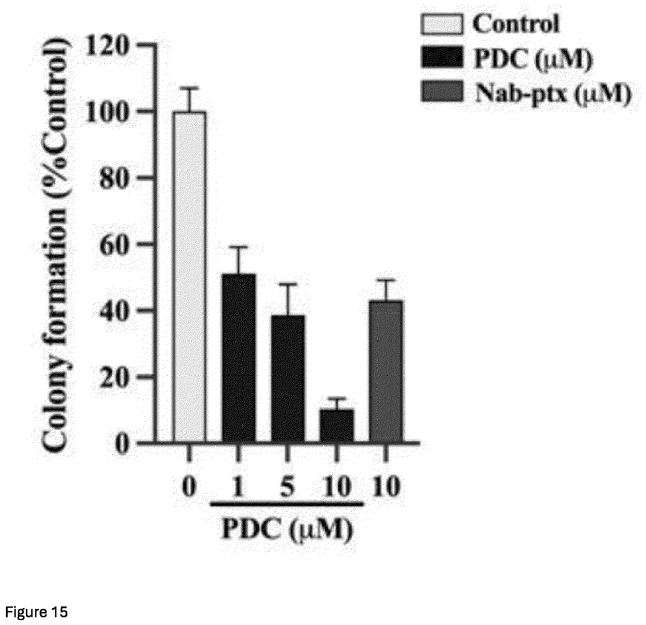

The results are shown in Figure 14 and Figure 15. The inhibition of targeted peptide modified nano paclitaxel polymer micelles on the growth of tumor cells was detected through the clone formation experiment. The results show that the targeted peptide modified nano paclitaxel polymer micelles can significantly inhibit the ability of pancreatic cancer cell cloning.

### Example 8

### sw1990 nude mouse model of pancreatic cancer

The specific operation is as follows: Take the logarithmic growth of 1 × 105 SW1990 cells were injected subcutaneously into the forelimbs of male B/C-nu mice.

On the 7th day, mice were randomly divided into four groups: control group, low dose group (10mg/kg/3day) of targeted peptide modified nano paclitaxel polymer micelles, high dose group (20mg/kg/3day), and albumin bound paclitaxel Nab PTX (20mg/kg/3day), with 6 mice in each group. When the subcutaneous tumor of the mouse is visible, measure the length (L) and width (W) of the tumor using a vernier caliper. Using the formula V=L × W2 × Calculate the tumor volume at 0.52. To test the toxicity of the drug, the body weight of mice is measured every three days. On the 35th day, all mice were killed. Detach the tumor in situ, measure the weight of the tumor in situ, and perform statistical analysis.

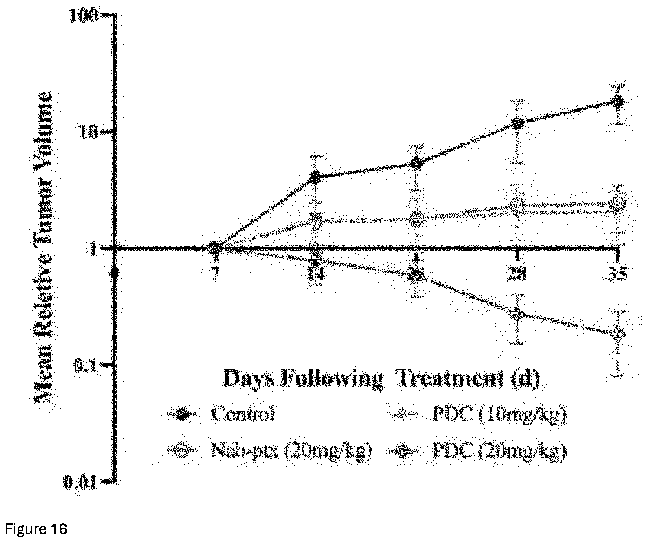

The results are shown in Figure 16. The sw1990 pancreatic cancer mouse model experiment shows that the targeted peptide modified nano paclitaxel polymer micelles also have good anti-tumor effect in animals. The tumor size of mice treated with targeted peptide modified nano paclitaxel polymer micelles (20mg/kg/3day) was significantly lower than that of the control group, and it was also significantly lower than the positive control albumin bound paclitaxel Nab PTX (20mg/kg/3day) group, indicating a significant tumor reduction effect. On the 35th day, the tumor in situ was dissected and the tumor volume was recorded in Figure 16.

## Claims

1. A nano drug delivery system (PDC) that specifically binds to the neurotensin receptor (NTR) through targeted peptide modification, wherein the nano drug delivery system is an amphiphilic block copolymer micelle that can specifically bind to the neurotensin receptors (NTR) through targeted peptide modification and the micelle is self-assembled through film hydration method;
the targeted peptide specifically binding to the neurotensin receptor (NTR) is composed of 14 amino acid sequences, with one end of the peptide being cysteine, which can specifically bind to micelles; this peptide can block the signaling pathway of the combination of NT and NTR, and inhibit the biological function produced by the combination of NT and NTR.

2. The nano drug delivery system according to claim 1, wherein the amphiphilic block copolymer comprises a maleimide terminal group, and the molecular weight of this polymer ranges from 2000 to 12000.

3. The nano drug delivery system according to claim 1, wherein the amphiphilic block copolymer contains or does not contain Fmoc lysine, with a molecular weight of 1000-8000.

4. The nano drug delivery system according to any one of claims 1-3, wherein the targeted peptide that can specifically bind to the neurotensin receptor (NTR) is modified into a polymer micelle by reacting with maleimide via thiol groups.

5. The nano drug delivery system according to claim 1, wherein the nano polymer micelle encapsulates anti-tumor drugs.

6. The method of preparing the nano drug delivery system according to claim 1, wherein it comprises the following steps:
step 1, prepare polyethylene glycol polylactide copolymer or polyethylene glycol polylactide Fmoc lysine copolymer and maleimide polyethylene glycol polylactide copolymer;
step 2, the copolymer and tumor therapeutic drugs are self-assembled into nano drug loaded polymer micelles through film hydration method;
step 3, the nano polymer micelles react with peptides to obtain targeted peptide modified nano drug loaded polymer micelles.

7. A method of treating tumor diseases expressing the neurotensin receptor (NTR), comprising the preparation of nano polymer micelles using the nano drug delivery system according to claim 1.
